Europäisches Patentamt

European Patent Office

Office européen des brevets

(19)

(11) Publication number: **0 330 891 B1**

# EUROPEAN PATENT SPECIFICATION

(12)

(45) Date of publication of patent specification: **25.11.92**  (51) Int. Cl.⁵: **G01L  19/00**, F16K 7/07, A61B 5/02

(21) Application number: **89102403.6**

(22) Date of filing: **11.02.89**

(54) An arrangement for pressure transmission.

(30) Priority: **03.03.88 SE 8800758**

(43) Date of publication of application:
**06.09.89 Bulletin  89/36**

(45) Publication of the grant of the patent:
**25.11.92 Bulletin  92/48**

(84) Designated Contracting States:
**BE CH DE ES FR GB IT LI NL**

(56) References cited:
**DE-U- 8 211 776**
**US-A- 2 972 464**
**US-A- 3 441 245**
**US-A- 4 268 005**

(73) Proprietor: **GAMBRO AB**
**Post Box 10101**
**S-220 10 Lund(SE)**

(72) Inventor: **Carlsson, Per-Olov Arne Vilhelm**
**Morkullevägen 11**
**S-280 10 Sösdala(SE)**

(74) Representative: **Boberg, Nils Gunnar Erik**
**Gambro AB Patent Department Box 10101**
**S-220 10 Lund(SE)**

EP 0 330 891 B1

## Description

### TECHNICAL FIELD

The present invention relates to an arrangement for pressure transmission, comprising an outer casing which by means of a membrane is divided into two separate spaces and which is provided with an inlet opening for the conducting of a first fluid to one of the said spaces and a connection opening for the connection of a second fluid from the other of the said spaces to a pressure sensing device, the membrane, preferably through hot-forming, being made to adhere tightly to at least a part of the periphery of the inlet opening in such a manner that the inlet opening communicates only with one of the two spaces, whilst the connection opening communicates only with the other of the two spaces.

The arrangement in accordance with the invention is intended in particular to be installed in a blood-transmitting duct for the monitoring of the blood pressure in connection with, for example, dialysis or any other extracorporeal blood treatment.

The term "fluid" is to be construed, though, in a wider sense comprising other liquids and also gases.

### BACKGROUND ART

The arrangement in accordance with the present invention may be said to constitute a further development of the pressure transmitting arrangement which is shown and described in more detail in the US patent 4 194 974. In this patent a pressure transmitting cushion 2 is provided in a blood-carrying duct 1 and can be connected to a pressure-sensing device via a duct 3. To prevent contamination of the pressure-sensing device, the known pressure-transmitting cushion is connected normally to the pressure-sensing device via a so-called disc filter. It is the object of the present invention to render unnecessary the use of such a disc filter. This would be feasible in the manner which is demonstrated in the US patent 3 863 504. However, this patent shows a very clumsy design which is difficult to produce and which, moreover, is suitable only for the measurement of negative pressure.

As regard the prior art reference is also being made to DE-U-82 11 776 and US-A-3 441 245.

### DISCLOSURE OF INVENTION

The present invention thus can be said to constitute a further development of the pressure cushion in accordance with the US patent referred to above firstly or refinement and further development of the pressure-transmitting arrangement in accordance with the US patent referred to above secondly. More particularly the invention relates to an arrangement for pressure transmission, comprising an outer casing which by means of a membrane is divided into two separate spaces and which is provided with an inlet opening for conducting a first fluid to one of the said spaces and a connection opening for the connection of the second fluid from the other of the said spaces to a pressure sensing device, the membrane, preferably through hot-forming, being made to adhere tightly to at least a part of the periphery of the inlet opening in such a manner that the inlet opening communicates with only one of the said spaces, whilst the connecting opening communicates only with the other of the two spaces. The arrangement according to the present invention is characterized in that the membrane is inserted in such a non-tensioned state, e g pleaded, that it can transmit positive as well as negative pressure. This design, as will be evident in more detail from the following, makes possible a very simple manufacturing process starting out from very simple blanks.

The invention thus can be applied to a design provided with only one inlet for the said first fluid. Preferably, however, the arrangement comprises also an outlet for this fluid, that is to say the arrangement is adapted so that this fluid flows through the same. In a preferred embodiment the arrangement here is formed of two blanks, namely an inner tubing of a pressure-transmitting membrane material and an outer pipe of a more rigid structure and provided with the said connection opening. The two ends of the tubing and of the pipe respectively can be made in a simple manner by hot-forming to adhere concentrically to each other to form the said inlet and outlet, which are made to communicate with only the interior of the tubing of membrane material, whilst the space outside the tubing is made to communicate with the said connection opening. If the primary object is to measure negative pressure the initial shape of the outer as well as of the inner blank may be chosen to be substantially circular-cylindrical. If on the other hand it is desired to measure positive pressure, the initial shape of the outer material may be chosen to be substantially circular-cylindrical, whilst the initial shape of the inner is chosen in the shape of a cylinder with pleats arranged in longitudinal direction.

If the two blanks are to be joined together by means of hot-forming, they ought to consist of materials which can be fused together and preferably of the same material.

In an alternative embodiment of the object of the invention the outer casing consists of two halves preferably adapted so that they can be joined together by means of hot-forming, which are combined with one another in such a manner that the membrane is inserted between them. The membrane is to be inserted in such a non-tensioned state, e g pleated, so that it can transmit positive as well as negative pressure.

The two housing halves preferably are manufactured in one piece held together by a hingeable connection about which they are folded to their ultimate position.

To facilitate the connection of the arrangement to a pressure-sensing device the said connecting opening may be in the form of a projecting nipple.

## BRIEF DESCRIPTION OF DRAWINGS

The invention will be described in more detail in the following with reference to the attached drawings which by way of example show some different preferred embodiments of the object of the invention.

Figures 1-3 show three outer views perpendicular to one another of a finished arrangement in accordance with the inventions.

Figures 4-6 show in corresponding line-up in two sections and one outer view two combined blanks, from which the arrangement in accordance with the figures 1-3 can be produced. In order to fall within the scope of the present invention the membrane shown has, however, to be arranged in such a non-tensioned state that it may be expanded out against the outer tube formed blank.

Figures 7-8 show two sections through the arrangement in accordance with figures 1-3 perpendicular to one another at a positive pressure in the inner tubing.

Figures 9 and 10 show the same sections at a negative pressure in the inner tubing.

Figures 11 and 12 show an alternative design of the inner membrane at pressure 0.

Figures 13 and 14 show in the same manner this alternative design at a positive pressure.

Figures 15 and 16 show in the same manner this alternative design at a negative pressure.

Figures 17 and 18 show two views perpendicular to one another of a finished arrangement in accordance with the invention in a further alternative embodiment. In order to fall within the scope of the present invention the membrane shown has, however, to be inserted in such a non-tensioned state that it can expand as well upwards as downwards from the position shown in figure 21.

Figures 19-21 show two sections perpendicular to one another and an outer view respectively of the blanks which are required for the manufacture of the arrangement in accordance with figures 17 and 18.

Figure 22 shows a section corresponding to that according to figure 21, but for an arrangement better designed for measurement of negative as well as positive pressure.

Figures 23 and 24 show designs in accordance with figures 21 and 22 in finished condition at pressure 0.

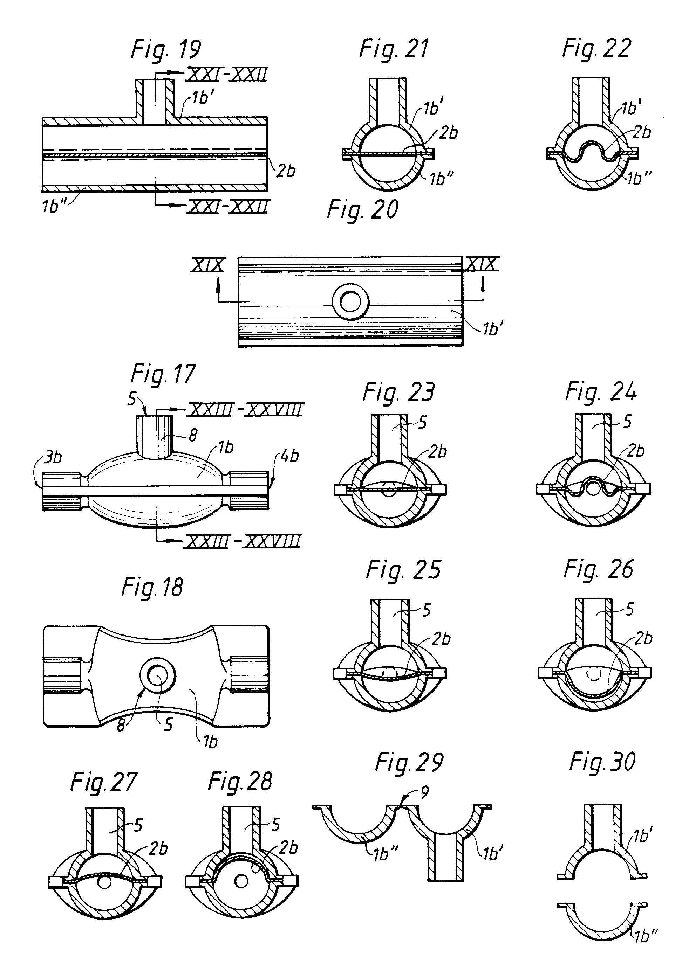

In the same manner figures 25 and 26 show the same designs at a negative pressure.

In the same manner figures 27 and 28 show the same designs on measuring a positive pressure.

Figures 29 and 30 finally show two further alternative embodiments of the blank for the outer casing forming part of the object of the invention.

## BEST MODE OF CARRYING OUT THE INVENTION

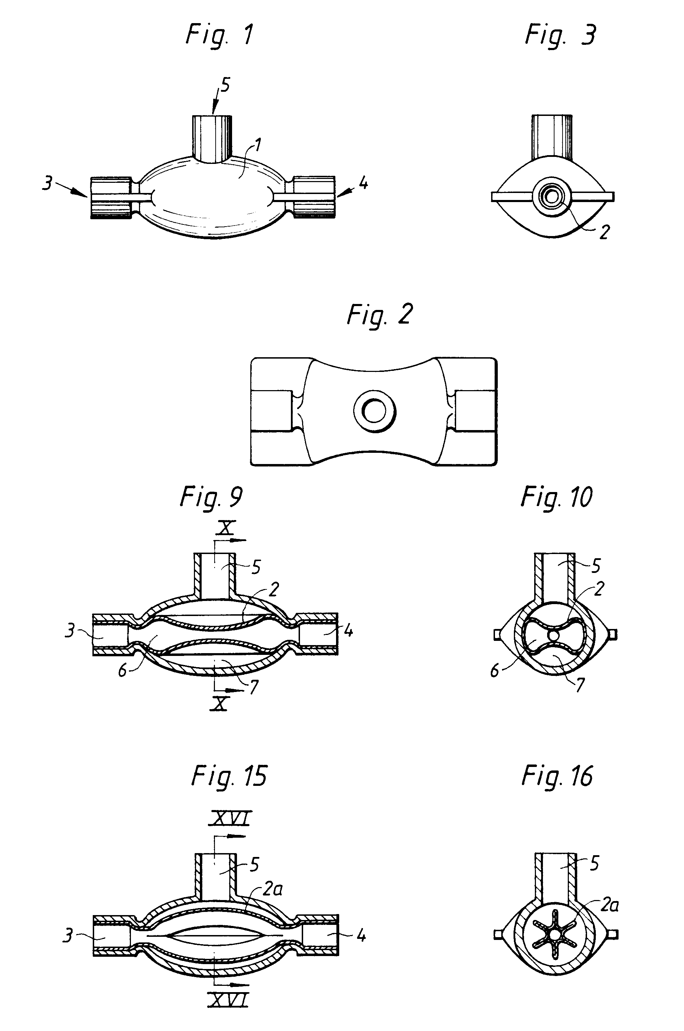

In fig 1-3 thus is shown a complete finished arrangement in accordance with the invention comprising an outer casing 1 and an inner membrane 2. The outer casing is provided with an inlet opening 3 and an outlet opening 4 and a connection opening 5 intended for connection to a pressure-sensing device, not shown on the drawing.

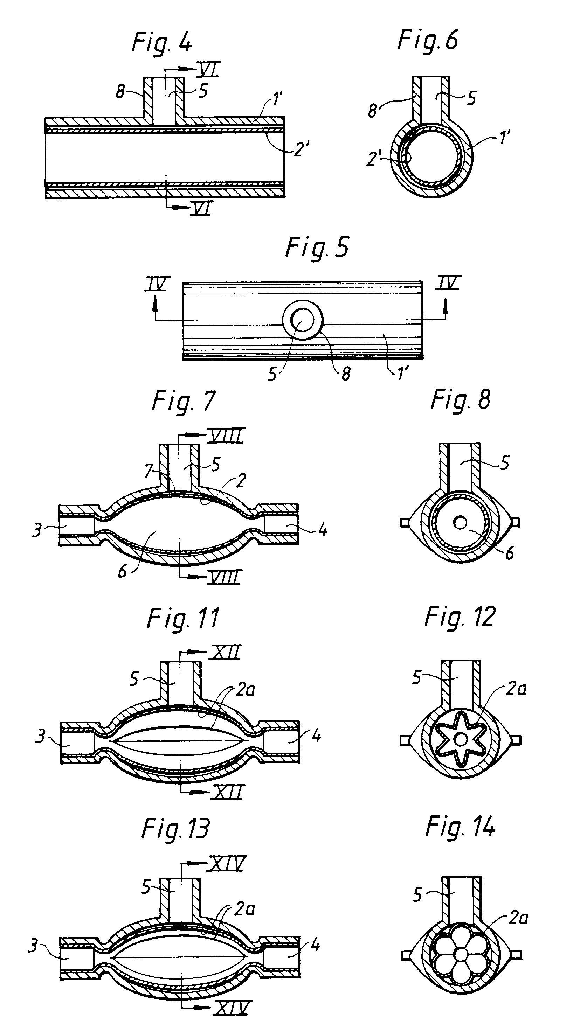

As is evident best from figures 7-10, the arrangement shown comprises an inner space 6 which communicates directly with the inlet opening 3 and the outlet opening 4. Moreover, there is a second space 7 outside the membrane 2 which is connected to the connection opening 5.

The figures 7 and 8 thus refer to the arrangement in neutral state, that is to say at pressure 0 in the inner space 3. If this pressure drops, the shape of the inner membrane 2 changes to that shown in figures 9 and 10. If larger positive pressures are to be measured the space 7 has to be enlarged compared with what is shown in figures 7 and 8.

The manufacture of the design described above starts out from the blanks shown in figures 4-6, that is to say from an inner substantially circular-cylindrical tubing of a membrane material which here is designated 2' and an outer pipe of a more rigid structure which here is designated 1'. The outer pipe 1' too is substantially circular-cylindrical at the same time as it is provided with an outer nipple 8 which comprises the connection opening 5. As will be seen readily from a comparison between the figures 4-6 and the figures 1-3 the blanks in accordance with the firstnamed figures can be converted easily by hot pressing to the shape shown in the figures 1-3. Preferably this is done by a fusing together of the material around the periphery of the inlet opening 3 and the outlet opening 4, respectively. The blanks thus have to consist of material which can be fused together and preferably of the same material. The conversion appropriately is done by using as a hold-on two mandrels which have an outer shape corresponding to the desired inner shape of the inlet opening 3 and of the outlet opening 4, respectively, and which extend a short distance into the space 6.

The figures 11 and 12, 13 and 14 and 15 and 16, respectively, show sections corresponding to figures 7 and 8 by way of an alternative design at pressure 0, at a positive pressure and at a negative pressure in the inner tubing. The sole difference here is that the inner tubing has pleats arranged in longitudinal direction. It has been given, therefore, in these figures the designation 2a. For the rest the design coincides with that described above, and for this reason the same reference designations have been used for the remaining parts.

In the figures 17 and 18 are shown two views perpendicular to one another of a further embodiment of the object of the invention. This embodiment is made up of the blanks shown in figures 19-21 which consist of an inner plane membrane 2b, an upper substantially semi-cylindrical casing part 1b' and a lower likewise substantially semi-cylindrical casing part 1b''. The membrane 2b thus is inserted in a non-tensioned state between the two outer casing parts 1b' and 1b'', at the same time as the whole design is formed to the shape shown in figures 17 and 18. In the process the membrane 2b is made to adhere tightly to the upper peripheral part of the inlet opening 3b and the outlet opening 4b, respectively. The connection opening 5 in the nipple 8 has not been modified and for this reason these reference designations have been retained in the figures 17 and 18.

As is evident from a comparison between the figures 21 and 22 the membrane 2b either can be inserted in a relatively plane state according to figure 21 or in the pleated state as in figure 22.

Figures 23 and 24, 25 and 26 and 27 and 28, respectively, show sections corresponding to those according to figures 21 and 22 at pressure 0, at a negative pressure and at a positive pressure, respectively.

Figures 29 and 30 finally show two further alternative embodiments of the blank for the outer casing which forms part of the object of the invention. In figure 29 this blanks consists of the two casing parts 1b' and 1b'' which have been injection-moulded in one piece held together by a hinge strip 9. In figure 30 the same parts are shown instead in the form of two separate pieces.

Naturally the invention is not limited solely to the embodiments described above, but may be varied within the framework of the subsequent claims. For example, the blank part 1b'', which is shown in figures 19, 21 and 22, may be modified in that it is designed in one piece with the membrane 2b shown in the same figures. This membrane may ge given either the plane form shown in figure 21 or the pleated form shown in figure 22. It will be evident to those versed in the art that further modifications too can be made. Many variations are conceivable, for example, of the star-shape shown in figures 12, 14 and 16. With regard to the dimensions of the initial blank, it can be said that the thickness of the membrane 2' appropriately is chosen in the order of magnitude 0.3-0.4 mm and the thickness of the outer blank 1' in the order of magnitude of 2 mm. These dimensions have proved suitable when PVC is used as a starting material. When a different material is used, the dimensions naturally are adapted to the elasticity and other properties of the same.

## Claims

1. An arrangement for pressure transmission comprising an outer casing (1) which by means of a membrane (2) is divided into two separate spaces (6,7) and which is provided with an inlet opening (3) for the conducting of a first fluid to one (6) of the said spaces (6,7) and a connection opening (5) for the connection of a second fluid from the other (7) of the said spaces (6,7) to a pressure-sensing device, the membrane (2), preferably through hot-forming, being made to adhere tightly to at least a part of the periphery of the inlet opening (3) in such a manner that the inlet opening (3) communicates only with one (6) of the two spaces (6 7), whilst the connection opening (5) communicates only with the other (7) of the two spaces, **characterized** in that the membrane (2b) is inserted in such a non-tensioned state, e.g. pleated, that it can transmit positive as well as negative pressure.

2. An arrangement in accordance with claim 1, comprising also an outlet (4) for the said first fluid, **characterized** in that it is formed by two blanks, namely an inner tubing (2') of a pressure-transmitting membrane material and of an outer pipe (1') of a more rigid structure and provided with the said connection opening (5), the two open ends of the tubing and the pipe respectively being made by hot-forming to adhere concentrically to each other to form the said inlet and outlet (3,4) which communicate only with the interior of the tubing (2) of membrane material, whilst the space outside the tubing (2) communicates with the said connection opening.

3. An arrangement in accordance with claim 2, **characterized** in that the initial shape of the outer blank (1') as well as of the inner (2') is substantially circular-cylindrical.

4. An arrangement in accordance with claim 2, **characterized** in that the initial shape of the outer blank (1') is substantially circular-cylindrical, whilst the initial shape of the inner is a cylinder (2a) with pleats arranged in longitudinal direction.

5. An arrangement in accordance with anyone of the preceding claims, **characterized** in that the two blanks (1',2') consist of material which can be fused together, and preferably of the same material.

6. An arrangement in accordance with claim 1, comprising also an outlet (4b) for the said first fluid, **characterized** in that the outer casing consists of two halves (1b',1b'') which can be joined together, preferably by means of hotforming, and which are combined with one another in such a manner that the membrane (2b) is inserted between them.

7. An arrangement in accordance with claim 6, **characterized** in that the two casing halves are manufactured in one piece held together by a hingeable connection (9) about which they are folded to their ultimate position.

8. An arrangement in accordance with anyone of the preceding claims, **characterized** in that the said connection opening (5) is in the form of a projecting nipple (8).

**Patentansprüche**

1. Einrichtung zur Druckübertragung mit einem Außengehäuse (1), welches durch eine Membran (2) in zwei getrennte Räume (6, 7) geteilt ist und welches mit einer Einlaßöffnung (3) für die Führung eines ersten Fließmittels zu einem (6) dieser Räume (6, 7) und einer Verbindungsöffnung (5) für die Verbindung eines zweiten Fließmittels von dem anderen (7) dieser Räume (6, 7) mit einer Druckabfühleinrichtung versehen ist, wobei die Membran (2), vorzugsweise durch Heißverformen, in solcher Weise zu einem dichten Anhaften an wenigstens einem Teil des Umfangs der Einlaßöffnung (3) gebracht ist, daß die Einlaßöffnung (3) nur in Verbindung mit einem (6) der beiden Räume (6, 7) steht, während die Verbindungsöffnung (5) nur in Verbindung mit dem anderen (7) der beiden Räume steht, **dadurch gekennzeichnet,** daß die Membran (2b) in solch einem nichtgespannten, z.B. gefalteten, Zustand eingesetzt ist, daß sie sowohl positiven als auch negativen Druck übertragen kann.

2. Einrichtung nach Anspruch 1 mit einem Auslaß (4) für das erste Fließmittel, **dadurch gekennzeichnet,** daß sie von zwei Teilen gebildet ist, nämlich einem Innenschlauch (2') eines druckübertragenden Membranmaterials und einem Außenrohr (1') einer steiferen Struktur, das mit der Verbindungsöffnung (5) versehen ist, wobei die beiden offenen Enden des Schlauches bzw. des Rohres durch Heißverformen zum konzentrischen Anhaften aneinander unter Bildung des Einlasses und des Auslasses (3, 4) gebracht sind, welche nur mit dem Inneren des Membranmaterialschlauches (2) in Verbindung stehen, während der Raum außerhalb des Schlauches (2) mit der Verbindungsöffnung in Verbindung steht.

3. Einrichtung nach Anspruch 2, **dadurch gekennzeichnet,** daß die Anfangsform des Außenteils (1') wie auch des Innenteils (2') im wesentlichen kreiszylindrisch ist.

4. Einrichtung nach Anspruch 2, **dadurch gekennzeichnet,** daß die Anfangsform des Außenteils (1') im wesentlichen kreiszylindrisch ist, während die Anfangsform des Innenteils ein Zylinder (2a) mit in Längsrichtung angeordneten Falten ist.

5. Einrichtung nach einem der vorausgehenden Ansprüche, **dadurch gekennzeichnet,** daß die beiden Teile (1',2') aus einem Material, das miteinander verschmolzen werden kann, und vorzugsweise aus dem gleichen Material bestehen.

6. Einrichtung nach Anspruch 1 auch mit einem Auslaß (4b) für das erste Fließmittel, **dadurch gekennzeichnet,** daß das Außengehäuse aus zwei Hälften (1b', 1b'') besteht, die vorzugsweise durch Heißverformen miteinander verbunden werden können und die miteinander derart kombiniert sind, daß die Membran (2b) zwischen ihnen eingesetzt ist.

7. Einrichtung nach Anspruch 6, **dadurch gekennzeichnet,** daß die beiden Gehäusehälften aus einem Stück gefertigt sind und durch eine Scharnierverbindung (9) aneinandergehalten werden, um welche sie in ihre Endstellung gebogen werden.

8. Einrichtung nach einem der vorausgehenden Ansprüche, **dadurch gekennzeichnet,** daß die Verbindungsöffnung (5) in der Form eines vorspringenden Nippels (8) vorliegt.

**Revendications**

1. Dispositif de transmission de pression comprenant un corps extérieur (1) qui est divisé par une membrane (2) en deux espaces séparés (6 ,7) et qui comporte un orifice d'entrée (3) pour l'amenée d'un premier fluide à un (6) desdits espaces (6,7) et un orifice de connexion (5) pour la connexion d'un deuxième fluide de l'autre (7) desdits espaces (6,7) à un dispositif de mesure de pression, la membrane (2) étant collée de façon étanche, de préférence par formage à chaud, à au moins une partie de la périphérie de l'orifice d'entrée (3) d'une manière telle que l'orifice d'entrée (3) communique seulement avec un (6) des deux espaces (6,7), tandis que l'orifice de connexion (5) communique seulement avec l'autre (7) des deux espaces, caractérisé en ce que la membrane (2b) est insérée dans un état non tendu, par exemple plissé, tel qu'elle peut transmettre aussi bien une pression positive qu'une pression négative.

2. Dispositif suivant la revendication 1, comprenant également une sortie (4) pour le dit premier fluide, caractérisé en ce qu'il est formé par deux ébauches, à savoir une chemise tubulaire intérieure (2') en matière de type membrane de transmission de pression et un tube extérieur (1') de structure plus rigide et comportant ledit orifice de connexion (5), les deux extrémités ouvertes de la chemise tubulaire et du tube respectivement étant collées mutuellement et concentriquement par formage à chaud pour définir ladite entrée et ladite sortie (3,4) qui communiquent seulement avec l'intérieur de la chemise tubulaire (2) en matière de type membrane, tandis que l'espace du côté extérieur de la chemise tubulaire (2) communique avec ledit orifice de connexion.

3. Dispositif suivant la revendication 2, caractérisé en ce que la forme initiale de l'ébauche extérieure (1') et de l'ébauche intérieure (2') est sensiblement cylindrique circulaire.

4. Dispositif suivant la revendication 2, caractérisé en ce que la forme initiale de l'ébauche extérieure (1') est sensiblement cylindrique circulaire tandis que la forme initiale de l'ébauche intérieure est un cylindre (2a) avec des plis disposés dans la direction longitudinale.

5. Dispositif suivant l'une quelconque des revendications précédentes, caractérisé en ce que les deux ébauches (1',2') sont en matières qui peuvent se souder l'une à l'autre, et de préférence en la même matière.

6. Dispositif suivant la revendication 1, comprenant également une sortie (4b) pour ledit premier fluide, caractérisé en ce que le corps extérieur est composé de deux moitiés (1b',1b") qui peuvent être assemblées, de préférence par formage à chaud, et qui sont combinées l'une à l'autre d'une manière telle que la membrane (2b) est insérée entre elles.

7. Dispositif suivant la revendication 6, caractérisé en ce que les deux demi-corps sont fabriqués en une seule pièce et sont maintenus ensemble par une jonction articulée (9) autour de laquelle ils sont pliés à leur position finale.

8. Dispositif suivant l'une quelconque des revendications précédentes, caractérisé en ce que le dit orifice de connexion (5) est sous la forme d'un mamelon en saillie (8).

Fig. 1

Fig. 3

Fig. 2

Fig. 9

Fig. 10

Fig. 15

Fig. 16

Fig. 4

Fig. 6

Fig. 5

Fig. 7

Fig. 8

Fig. 11

Fig. 12

Fig. 13

Fig. 14

8

Fig. 19

XXI-XXII

1b'

2b

1b''

XXI-XXII

Fig. 21

1b'

2b

1b''

Fig. 22

1b'

2b

1b''

Fig. 20

XIX

XIX

1b'

Fig. 17

5

XXIII-XXVIII

8

1b

3b

4b

XXIII-XXVIII

Fig. 23

5

2b

Fig. 24

5

2b

Fig. 18

8

5

1b

Fig. 25

5

2b

Fig. 26

5

2b

Fig. 27

5

2b

Fig. 28

5

2b

Fig. 29

9

1b''

1b'

Fig. 30

1b'

1b''

9